Europäisches Patentamt

European Patent Office

Office européen des brevets

(19)

(11) Publication number: **0 216 042 B1**

## EUROPEAN PATENT SPECIFICATION

(12)

(45) Date of publication of patent specification: 06.03.91    (51) Int. Cl.⁵: **A61M 1/12**

(21) Application number: 86109458.9

(22) Date of filing: 10.07.86

(54) Cardiac assist device.

(30) Priority: 01.08.85 US 761252

(43) Date of publication of application:
01.04.87 Bulletin 87/14

(45) Publication of the grant of the patent:
06.03.91 Bulletin 91/10

(84) Designated Contracting States:
**DE FR NL**

(56) References cited:
**DD-A- 218 558**
**US-A- 4 078 267**
**US-A- 4 302 854**

**SURGICAL FORUM 9, 1959 A. KANTROWITZ,
W. MCKENNON "The experimental Use of the
Diaphragm as an Auxilliary Myocardium"
Pages 266-268**

**THE JOURNAL OF THORACIC AND CARDIO-
VASCULAR SURGERY, vol. 87, no. 3, March
1984 M.L. DEWAR et al. "Synchronously
stimulated Skeletal Muscle Craft for myocar-
dial Repair" pages 325-331**

**THE JOURNAL OF SURGICAL RESEARCH,
vol.4, no. 1, January 1964 K. NAKAMURA, W.**

GLENN "Craft of the Diafhragm as a Func-
tioning Substitut for the Myocardium" pages
435-439

(73) Proprietor: **MEDTRONIC, INC.**
**7000 Central Avenue N.E.**
**Minneapolis Minnesota 55432(US)**

(72) Inventor: **Khalafalla, Aida S.**
**2551 37th Avenue South**
**Minneapolis Minnesota 55406(US)**

(74) Representative: **Kopacz, William James**
**83, Avenue Foch**
**F-75116 Paris(FR)**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid (Art. 99(1) European patent convention).

Rank Xerox (UK) Business Services

## Description

BACKGROUND OF THE INVENTION

The present invention relates to a totally implantable muscle-powered cardiac assist device to be used as an auxiliary pump in conjunction with the natural heart. In one configuration the device comprises a pair of tubular shunts coupled to the aorta and left ventricle of the heart which communicate with an elastic chamber formed in the shape of an oblate ellipsoid. Valves located within the shunts permit blood to flow from the weak or damaged left ventricle of the heart into the aorta when the elastic chamber is compressed. An alternate configuration involves the use of the elastic chamber as an extra-aortic counterpulsation device with no valve requirement.

The mechanical energy required to compress the chamber is supplied by an innervated autogenous muscle surrounding the elastic chamber. This muscle is stimulated by an implantable pulse generator in synchrony with the ventricular depolarization of the patient's heart. In operation, the contraction of the elastic chamber under the influence of a muscle tissue forces blood into the aorta. Additionally, the pulse generator provides chronic ultra-low frequency stimulation to the muscle tissue to maintain a high population of slow twitch-type muscle fibers.

The use of autogenous muscle to drive mechanical pumps is known in the art from U.S. Patent No. 4,078,267 which discloses an artificial heart propelled by respiratory muscles. Devices of this type have enjoyed only limited success because, mammalian skeletal muscle is not capable of long-term pumping due to metabolic fatigue. Recently it has been demonstrated that chronic electrical stimulation of muscle tissue produces an adaptive transformation of muscle tissue which increases the capillary density in the muscle tissue as well as the mytochondrial volume and results in an increased work capacity of the transformed muscle. Histologically, such tissue is transformed to the slow twitch-type which exhibits greatly increased resistance to fatigue.

Early experimental evaluation of skeletal muscles for myocardial augmentation was reported by Kantrowitz and McKennon. See Experimental Use of the Diaphragm as an Auxiliary Myocardium, Surgical Forum 9, Page 266, 1959. By wrapping diaphragm muscle around the heart and stimulating it via the phrenic nerve, they observed no significant hemodynamic effects; however, when employed as the counterpulsation device, they noted a short-term increase in the diastolic aortic pressure. Later, in 1964, Nakamora and Glenn utilized the diaphragm to assist atrial function. The diaphragm graft in the atrium continued to contract in response to stimulation from the phrenic nerve and served to elevate the right atrial pressure chronically. See Graft of the Diaphragm as a Functioning Substitute for the Myocardium; an Experimental Study , J Surg Res 4; 435, 1964.

Other approaches which involve the use of small spring-loaded diaphragm pumps with externally positioned flap valves have been energized by canine quadricept femorous muscles. Mechanical pumps of this type have shown outputs of 600-700 milliliters per minute.

These early studies demonstrated the potential for the use of skeletal muscles to augment ventricular action of the heart. However, this initial work indicated that a critical problem existed in the deterioration of muscle performance with continued use. Attempts at improving the hemodynamic behavior of the muscle graft by lower frequency stimulation was demonstrated by Doer, et al in 1984. See Synchronously Stimulated Skeletal Muscle Graft for Myocardial Repair, J Thorac Cardiovasc Surg 87: 325, 1984. These more recent studies demonstrated that skeletal muscle, while initially capable of hemodynamic work, fatigues rapidly even under conditions less demanding than those which are tolerated indefinitely by the cardiac muscle itself.

Although skeletal muscles contain populations of fibers which share many of the characteristics of cardiac muscle tissue, the skeletal type (I) or slow twitch fibers serve primarily a postural role in that they are required to sustain prolonged periods of activity without appreciable fatigue. However, in the tissue suitable for application to cardiac assist devices, these fibers are interspersed with at least an equal number of fast or type (II) fibers. These latter fibers have the properties suited to brief periods of intense activity, their fast contractile characteristics derive from specific contractile protein isoforms and extensive sacrotubular system and their dependence on energy derived from anaerobic glycolysis. This metabolic substrate renders the muscles susceptible to fatigue under conditions of prolonged use even at low cardiac rate duty cycles such as those demonstrated by Doer. Additionally, unlike cardiac muscle cells which contract as a synctyium, skeletal muscle fibers are normally recruited to an extent determined by the intensity of activation and in a fixed sequence. In practice, the fast fibers are the first to contract and the slow fibers are the last to contract. This structural property of the skeletal muscles minimizes the functional demand placed upon the fibers which are most susceptible to fatigue. However, the application of such tissues to cardiac assist devices require that all of the muscle tissues be recruited simultaneously and be equally active with the consequence of chronic fatigue.

Over the past fifteen years, however, a plasticity of muscle fiber type has been demonstrated in response to chronic electrical stimulation. In 1969, Salmons, et al demonstrated that the contractile speed of fast muscles could be modulated to a striking extent by continuous electrical stimulation of the motor nerve at a frequency of 10 Hz.

There is now a large body of evidence to show that fast skeletal muscles can ultimately acquire all of the physiological, biochemical, and ultrastructural characteristics of slow muscle under conditions of chronic stimulation. Such adapted muscles demonstrate a corresponding increase in the use of enzymes for aerobic metabolism and a decrease in the enzymes for glycolysis.

When a change is also involved, the contractile proteins period is reflected by an increased conversion of light to heavy chain insoforms of myosin characteristic of slow muscle tissue. As these changes progress over a period of months, the muscle mass contracts progressively more slowly and is more resistant to fatigue than initially. These recent developments have suggested that appropriately adapted skeletal muscle may be harvested to restore myocardial function through surgical procedures.

In the present application, however, chronically stimulated and transformed muscle tissue is utilized to actuate a biological pump implanted within the body and connected to the aorta for assisting a weakened or diseased ventricle in the delivery of blood to body tissues.

In this context, the present invention is directed to an optimized biological pump which exploits the ability of transformed tissue to augment the ventricular action of the heart. This invention discloses two alternate embodiments to achieve the desired goal of a totally implantable, body-compatible cardiac assist system.

According to another prior art device, DD 218558 A1, there is seen a device for stimulating the breast muscle causing a contraction of this muscle. Under this muscle, is an elastic chamber which is compressed, this intended to function as an artificial heart. There is, however, no timing interaction between this artifical heart and the patient's heart, the artificial heart being stimulated at a fixed rate. Further, there are no means for conditioning the muscle which surrounds the chamber.

According to the invention, however, there is provided a means for pumping blood from the left ventricle to the aorta of the patient's heart in synchrony with the ventricular depolarizations. This pumping means includes a first tubular shunt having a proximal and a distal end for connection to the left ventricle of the heart through the proximal end a second tubular shunt having a proximal and distal end for connection to said aorta of said heart. The elastic pumping chamber has an oblate ellipsoidal shape defined by a major axis and a minor axis adapted to be substantially completely surrounded by a sheath of innervated autogenous muscle tissue and also has first and second aperatures located at the periphery concentric with the major axis. There is also provided a unidirectional aortic valve coupled to said distal end of the first tubular shunt and coupled to said first aperture and a unidirectional mitral valve coupled to said distal end of the second tubular shunt and coupled to said second aperture. In addition, there is pulse generator adapted to be coupled to a first and a second electrode for providing chronic stimulation pulses to muscle tissue at a frequency , called ultra-low frequency, low enough to maintain a high population of slow twitch type muscle fibers and also for providing the ventricle at an amplitude such as to stimulate said muscle tissue in order to compress said pumping chamber. The first electrode is adapted to be coupled to the autogenous muscle tissue for stimulation and to be coupled to the pulse generator while the second electrode is adapted to be coupled to the ventricle for sensing depolarization of ventricular tissues.

According to another embodiment of the invention, there are provided means for augmenting blood flow from the left ventricle to the aorta in synchrony with the ventricular repolarization of the patient's heart, which includes an elastic spherical pumping chamber surrounded by a sheath of innervated autogenous muscle tissue with the chamber being adopted to be located in the transected aorta. There is also provided a pulse generator adapted to be coupled to a first and a second electrode wherein the first electrode is adapted to be coupled to said autogenous muscle tissue for stimulation and adapted to be coupled to said pulse generator whereby the pulse generator provides chronic stimulation to the muscle tissue at a frequency, called the ultra-low frequency, low enough to maintain a high population of slow twitch type muscle fibers and wherein the second electrode is adapted to be coupled to said ventricle for sensing end repolarizations of ventricle tissues.

Variations and further specifics of this invention are set forth in the dependent claims.

Brief Description of the Drawings

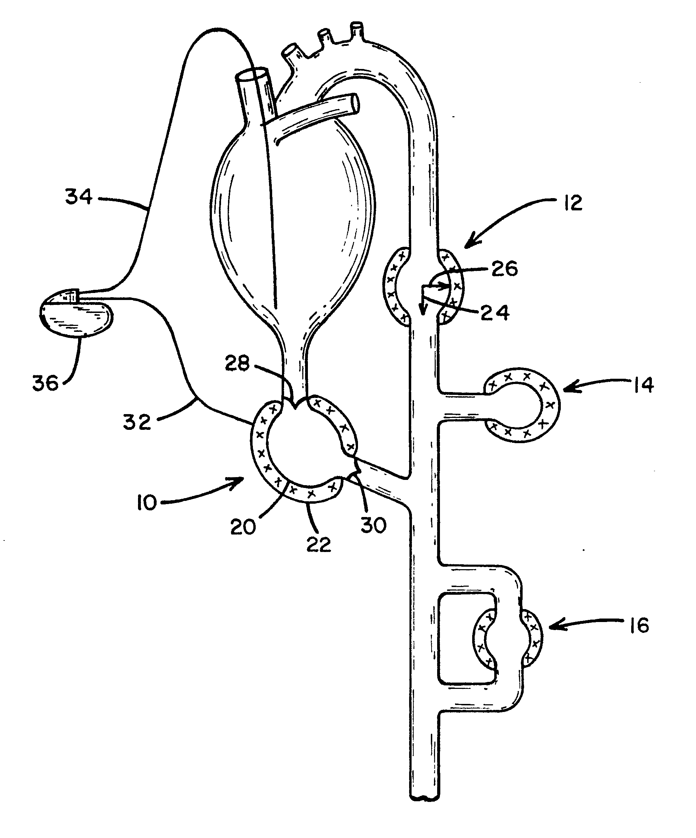

The figure shows a cross-section of the cardiac assist device of the AACS system as well as a cross-section of the EABC system.

Detailed Description of the Invention

I. The first embodiment is referred to as a Apico-Aortic Conduit System (AACS), depicted in the Figure at 10; and, the second embodiment is referred to as an Extra-Aortic Balloon Counterpulsation System (EABC) and is shown in the alternative attachments to the descending aorta at 12, 14 and 16.

In either embodiment, the pump consists of an elastomeric chamber 20, surrounded by a muscle sheath 22, formed from transformed muscle tissue. The chamber is shaped in the form of an oblate ellipsoid having a horizontal axis 26 and a vertical axis 24. In the AACS system, unidirectional heart valves 28, 30 may be provided to establish the flow direction of blood through the chamber. These valves are located in apertures formed in the periphery of the elastomeric chamber. Valves suitable for this application include the Medtronic mitral heart valve Model 7700 having an orifice diameter of 2 cm for the entry valve 28. A valve suitable for the exit valve 30 of the chamber is the Medtronic aortic heart valve Model A7700 having an orifice diameter of 1.6 cm.

The elastic chamber is shaped in the form of an ellipsoid of revolution. The generating ellipse has a major or horizontal axis 26, which is the axis of revolution and a minor or vertical axis 24 as shown in the Figure. For a desired fluid stroke volume of 70 cc, the chamber should have a volume of approximately 140 cc. This is based upon an assumed ejection ratio of 50%. For a volume of 140 cc, the dimensions of the major and minor axes are related by $b = 5.78/\sqrt{a}$.

To compute the minimum force required to pump the desired stroke volume, one may model the chamber as an equivalent cylinder, having a volume equal to the chamber, whose length is equal to the horizontal axis of the ellipse. In this instance, the cylinder will have a base radius $\bar{b}$ given by $\bar{b} = 4.72/\sqrt{a}$. The force required to displace the desired blood volume is given by:

$$(70/\tau\tau\bar{b})^2 \, (\rho \, /\pi) = 602.95a$$

where $\tau$ is the ejection time (0.35 sec) and $\rho$ is the specific gravity of blood (1.055). This force corresponds to the end pressure or terminal pressure, $P_{ter}$, in the chamber distributed over the exit aperture of the chamber as determined by the size of the aortic valve aperture, $r_o$.

In practice, sufficient muscle mass is wrapped around the balloon to generate a static pressure of 120 mm of mercury or $1.6 \times 10^5$ dynes per square, centimeter within the chamber. This is the available pressure, $P_{avl}$, responsible for driving blood into the body systemic vessels.

The mass flow rate for a Newtonian fluid in the laminar regime is given by Poiseuille expression

$$f = \frac{\pi P r^4}{8L \eta}, \text{ where P is the pressure, L is the length of}$$

the tube, r is the tube radius, and $\eta$ is the viscosity coefficient. As previously mentioned, the available pressure responsible for driving the fluid out of the pump is related to the radius of the aortic valve as indicated by the relationship above. Likewise, the minimum pressure or terminal pressure in the chamber is related to the average radius of the balloon which is taken as the radius of the equivalent cylinder. The quantity $Pr^4$ in the Poiseuille relation gives an estimate of the system compliance, and therefore, to achieve maximum compliance matching, we should have

$P_{avl} \times r^4 = P_{ter} \times (\bar{b})^4$. This leads to: a $r_o^6 = 0.595$. For an aortic valve orifice, $r_o$, of 0.8 cm, we have a = 2.27 cm and b = 3.84 cm for the desired dimensions of the oblate ellipsoid.

Optimization of the chamber size is based on a fluid flow rate, f, expressed in cc's per second, which is equal to the systolic's cardiac output of the cardiac assist device. The parameters should be optimized to provide a stroke volume of 70 cc, an ejection time of 350 ms and a volume flow rate of 200 cc per second. The fluid velocity is given by the flow rate divided by the cross-sectional area, A. Therefore, the average flow velocity during systolic time, $\bar{v} = f/\bar{A} = 2.86a$. At the end of the ejection time, the fluid flow velocity within the chamber must become zero.

With respect to the muscle mass 22 required in this cardiac assist device, one can use Young-LaPlace equation to compute the tension required at the wall of the chamber to generate the 120 mm of mercury pressure. For a cylindrical balloon of unit radius, the wall tension is computed to be $1.6 \times 10^5$ dyne per centimeter. Measurements of muscle fibers reveal that the isometric force generated by a tensed muscle is

approximately $2.9 \times 10^3$ grams per square centimeter of muscle cross-section or $2.9 \times 10^6$ dynes per square centimeter of muscle cross section. See Casey, E.J.; "Biophysics, Concepts and Mechanisms," Reinhold Books, New York, 1962, p 293. Calculations for a cylindrical balloon of unit radius (R = 1 cm) and sufficient length to accommodate at least 70 ml of blood leads to the following two useful rules of thumb:

Rule 1: $M = 2 \times 10^{-3} R P$

Where M is the muscular mass in grams, R is the balloon or bladder radius in cm, and P is the balloon pressure in dynes/cm$^2$.

Rule 2: $r_0{}^6 R^{-2} P = 4 \times 10^3$

where $r_0$ is the radius of the tube connecting the balloon to the aorta.

For example, in order to achieve the human systolic pressure of 120 mm Hg ($1.6 \times 10^5$ dynes/cm$^2$) in a 70 ml balloon of radius R = 1 cm, the required muscle mass is about 320 gm (11.3 oz.) according to Rule 1. Also, the radius, $r_0$ of the aortic valve, is estimated as 0.5 cm (0.2 inch from Rule 2).

By imposing an $r_0$ value of 0.8 cm and ejection ratio of 50% on the design parameters, it can be shown that the muscle mass required to wrap around the two caps of the oblate ellipsoid of volume 140 cm$^3$ is approximately twice that required for a volume of 70 cm$^3$, i.e., 645 gm (23 oz.)

II--The Extra-Aortic Balloon Counterpulsation Pump (EABC)

The pumping chamber here needs no entrance or exit valves as shown in the Figure at 12, 14 and 16. The EABC chamber is connected directly to the divided left subclavian artery distal to the thoracodorsal and thoracoacromial branches. A series (T-connection) 14 or parallel (U-connection) 12, 16 pump can be used. The T-connection is shown in the Figure. The balloon can either be wrapped by the rectus abdominus and latissimus dorsi pedicles, or placed deep to the pectoralis major.

The powering muscle would be stimulated directly by two wire electrodes 32. The stimulator is triggered from the left ventricular electrocardiogram via lead 34 or from the arterial pressure tracing output. Unlike the AACS, in this embodiment the pump would be triggered at the end diastolic phase of the cardiac cycle. This allows increased muscle perfusion which occurs while the muscle is relaxed during systole. Thus, fatigue can be considerably minimized, not only by this operational mode, but also by using the optimal stimulation parameters and protocol as with the AACs.

In addition, the hemodynamic requirements for the EABC device are minimal. There are no valve requirements and the balloon volume can be chosen commensurate with the severity of the situation. A balloon volume of 30 to 70 cc is recommended with an optimum size of 50 cc. The only requirement is that the balloon shape be spherical or nearly spherical in order to avoid sharp edges and corners where blood may stagnate.

The EABC system can be made to offset the primary or essential hypertension. This type of high blood pressure is caused by the progressive increase in construction of arteries and arterioles and their decreasing compliance, a phenomena which gradually increases with age. This is to be distinguished from malignant hypertension which arises from hormonal disturbances of the adrenal glands that sit atop of the kidneys or from malfunctioning of the baroreceptors of the carotid sinus which is in the back of the neck.

By adjusting the pressure wave on the extra-aortic balloon, one can augment the systolic pressure by decreasing the diastolic pressure level. Notice that infants average 80/46 in blood pressure at birth which rises to 100/60 during the first ten days, and levels up at 120/70 during adulthood. The following increase seems to be gradual reaching 135/80 in the fifties and 150/85 in the seventies. The borderlines of 160/95 are at best empirical in the sense that they represent a gradual process, and a 50 year-old subject with 160/90 blood pressure is the equivalent of a healthy counterpart who was 135/80 in his fifties and would extrapolate to 160/90 at 90 or 100 years. The invention disclosed herein involves the gradual augmentation of the cardiac output in such a way to compliantly meet this progressive imbalance with no extra demand from the heart muscle itself.

The pulse generator 36 of the present device must be adapted to provide chronic background stimulation to the innervated autogenous muscle tissue to provide for the maintenance of a high type two fiber population.

To provide for optimization of the stimulation parameters for any given individual it is required that the pulse generator be capable of providing burst stimulation with a burst duration between 150 and 500 milliseconds, with a number of pulses in a burst being less than or equal to 20. The pacemaker should also be capable of providing stimulation pulses at a rate between 0 and 150 beats-per-minute with a pulse width duration of between 150 and 500 microseconds. To provide for adjustable thresholds of the autogenous tissue, it is desirable to have an amplitude adjustable within the range of 0 to 15 volts with constant current output. The device must have an R-wave synchronous or triggered operating mode for stimulating the autogenous muscle in phase with the depolarization of the cardiac tissue for use in configuration 10. The delay from ventricular sense to stimulus should be variable between 20 and 500 milliseconds and be programmable by the attending physician.

It may also be desirable to provide for stimulating the autogenous tissue at a rate proportional to the sinus rhythm of the patient.

## Claims

1. A cardiac assist device including an elastic pumping chamber (20) adapted to be assisted by muscle (22), the device including tubular shunts connected to the chamber and valves coupled to the shunts and further including electrodes connected to an electrical power source and to said muscle,

    the device characterized in that there are included

    means for pumping blood from the left ventricle to the aorta of a patient's heart in synchrony with the ventricular depolarizations, said pumping means including,

    a first tubular shunt having a proximal and a distal end for connection to the left ventricle of the heart through the proximal end;

    a second tubular shunt having a proximal and distal end for connection to said aorta of said heart;

    said elastic pumping chamber (20) having an oblate ellipsoidal shape defined by a major axis (26) and a minor axis (24) adapted to be substantially completely surrounded by a sheath of innervated autogenous muscle tissue (22), and having first and second aperatures located at the periphery concentric with the major axis;

    a unidirectional aortic valve (28) coupled to said distal end of the first tubular shunt and coupled to said first aperture;

    a unidirectional mitral valve (30) coupled to said distal end of the second tubular shunt and coupled to said second aperture;

    a pulse generator (36) adapted to be coupled to a first (32) and a second (34) electrode
    - for providing chronic stimulation pulses to muscle tissue at a frequency, called ultra-low frequency, low enough to maintain a high population of slow twitch type muscle fibers;
    - and also for providing stimulation pulses in synchrony with detected depolarizations of the ventricle at an amplitude such as to stimulate said muscle tissue (22) in order to compress said pumping chamber (20);

    the first electrode (32) being adapted to be coupled to the autogenous muscle tissue for stimulation and being coupled to the pulse generator (36)

    the second electrode (34) being adapted to be coupled to the ventricle for sensing depolarization of ventricular tissues.

2. The device of claim 1 wherein the chamber (20) has a volume greater than 100 ml but less than 200 ml.

3. The device of claim 1 wherein the first tubular shunt has an internal diameter between 1.5 cm and 2.5 cm.

4. The device of claim 1 wherein the second tubular shunt has an internal diameter between 1.1 cm and 2.1 cm.

5. The device of claim 1 wherein the length of the vertical minor axis (b) and horizontal axis (a) bear the relationship: $b = 5.78/a^{1/2}$

6. The device of claim 1 wherein the muscle mass, m, required to wrap the chamber is greater than 350 gm and less than 600 gm.

7. The device of claim 1 wherein the ultra-low frequency stimulation pulses are square waves and are produced at a frequency greater than 20 Hz and less than 50 Hz, with a preferred frequency of 35 Hz.

8. A cardiac assist device including an elastic pumping chamber (20) adapted to be assisted by muscle (22), the device including electrodes connected to an electrical power source and to said muscle,

the device characterized in that there are included

means for augmenting blood flow from the left ventricle to the aorta in synchrony with the ventricular repolarization of the patient's heart, said means including:

an elastic spherical pumping chamber (12, 14, 16) surrounded by a sheath of innervated autogenous muscle tissue (22);

said chamber adapted to be located in the transected aorta;

a pulse generator (36) adapted to be coupled to a first (32) and a second (34) electrode;

wherein the first electrode is adapted to be coupled to said autogenous muscle tissue for stimulation and adapted to be coupled to said pulse generator whereby the pulse generator provides chronic stimulation to the muscle tissue at a frequency, called ultra-low frequency, low enough to maintain a high population of slow twitch type muscle fibers;

and wherein the second electrode is adapted to be coupled to said ventricle for sensing end repolarizations of ventricle tissues.

9. The device according to claim 8 wherein the chamber is connected to the transected aorta by a T connection.

10. The device according to claim 8 wherein the chamber is connected to the transected aorta by a U connection.

11. The device according to claims 8, 9, or 10 wherein the chamber has a volume between 30 and 70 ml and most preferably 50 ml.

12. The device according to claims 8, 9, 10, or 11 wherein counterpulsations are used to offset primary or essential hypertension.

**Revendications**

1. Dispositif d'assistance cardiaque incluant une chambre élastique de pompage (20) apte à être assistée par un muscle (22), le dispositif incluant des dérivations tubulaires raccordées à la chambre, et des soupapes accouplées aux dérivations, et incluant en outre des électrodes raccordées à une source d'énergie électrique et audit muscle,

le dispositif étant caractérisé en ce qu'il inclut des moyens pour pomper le sang depuis le ventricule gauche en direction de l'aorte du coeur d'un patient en synchronisme avec les dépolarisations ventriculaires, lesdits moyens de pompage incluant

une première dérivation tubulaire possédant une extrémité proximale et une extrémité distale pour le raccordement au ventricule gauche du coeur, par l'intermédiaire de l'extrémité proximale;

une seconde dérivation tubulaire possédant une extrémité proximale et une extrémité distale pour le raccordement à ladite aorte dudit coeur;

ladite chambre élastique de pompage (20) possédant une forme ellipsoïdale aplatie définie par un grand axe (26) et un petit axe (24) et apte à être entourée sensiblement complètement par une gaine d'un tissu musculaire autogène innervé (22), et possédant des première et seconde ouvertures situées sur la périphérie, concentriquement au grand axe;

une soupape aortique unidirectionnelle (28) accouplée à ladite extrémité distale de la première dérivation tubulaire et accouplée à ladite première ouverture;

une soupape mitrale unidirectionnelle (30) accouplée à ladite extrémité distale de la seconde dérivation tubulaire et accouplée à ladite seconde ouverture;

un générateur d'impulsions (36) adapté pour être accouplé à une première électrode (32) et à une seconde électrode (34)

- pour appliquer des impulsions de stimulation chronique au tissu musculaire à une fréquence,

7

désignée sous le terme de fréquence ultra-basse, suffisamment basse pour entretenir une population élevée de fibres musculaires du type à contraction lente;
- et également pour appliquer des impulsions de stimulation en synchronisme avec des dépolarisations détectées des ventricules, avec une amplitude apte à stimuler ledit tissu musculaire (22) pour comprimer ladite chambre de pompage (20); la première électrode (32) étant adaptée pour être accouplée au tissu musculaire autogène pour la stimulation et étant accouplée au générateur d'impulsions (36), la seconde électrode (34) étant adaptée pour être accouplée au ventricule pour détecter une dépolarisation de tissus ventriculaires.

2. Dispositif selon la revendication 1, dans lequel la chambre (20) possède un volume supérieur à 100 ml, mais inférieur à 200 ml.

3. Dispositif selon la revendication I, dans lequel la première dérivation tubulaire possède un diamètre intérieur compris entre 1,5 cm et 2,5 cm.

4. Dispositif selon la revendication 1, dans lequel la seconde dérivation tubulaire possède un diamètre intérieur compris entre 1,1 cm et 2,1 cm.

5. Dispositif selon la revendication 1, dans lequel les longueurs du petit axe vertical (b) et de l'axe horizontal (a) satisfont à la relation $b = 5,78/a^{1/2}$.

6. Dispositif selon la revendication 1, dans lequel la masse musculaire m nécessaire pour envelopper la chambre est supérieure à 350 g et inférieure à 600 g.

7. Dispositif selon la revendication 1, dans lequel les impulsions de stimulation à fréquence ultra-basse sont des ondes carrées et sont produites à une fréquence supérieure à 20 Hz et inférieure à 50 Hz, avec une fréquence préférée égale à 35 Hz.

8. Dispositif d'assitance cardiaque incluant une chambre élastique de pompage (20) adaptée pour être assistée par un muscle (22), le dispositif incluant des électrodes raccordées à une source d'énergie électrique et audit muscle,
le dispositif étant caractérisé en ce qu'il comporte des moyens pour augmenter le flux sanguin depuis le ventricule gauche en direction de l'aorte en synchronisme avec la repolarisation ventriculaire du coeur du patient, lesdits moyens incluant :
une chambre élastique sphérique de pompage (12,14,16) entourée par une gaine d'un tissu musculaire autogène innervé (22);
ladite chambre étant adaptée pour être située dans l'aorte coupée transversalement;
un générateur d'impulsions (36) adapté pour être accouplé à une première électrode (32) et à une seconde électrode (34);
la première électrode étant adaptée pour être accouplée audit tissu musculaire autogène pour la stimulation et étant adaptée pour être accouplée audit générateur d'impulsions; grâce à quoi le générateur d'impulsions applique une stimulation chronique au tissu musculaire à une fréquence, désignée sous le terme de fréquence ultra-basse, suffisamment basse pour entretenir une population élevée de fibres musculaires du type à contraction lente; et la seconde électrode étant adaptée pour être accouplée audit ventricule pour détecter des repolarisations finales de tissus ventriculaires.

9. Dispositif selon la revendication 8, dans lequel la chambre est raccordée à l'aorte coupée transversalement, par un raccord en T.

10. Dispositif selon la revendication 8, dans lequel la chambre est raccordée à l'aorte coupée transversalement par un raccord en U.

11. Dispositif selon les revendications 8,9 ou 10, dans lequel la chambre possède un volume compris entre 30 et 70 ml et égal de façon plus préférentielle à 50 ml.

12. Dispositif selon les revendications 8,9,10 ou 11, dans lequel des contre-pulsations sont utilisées pour compenser une hypertension primaire ou essentielle.

**Ansprüche**

1. Vorrichtung zur Herzunterstützung mit einer von einem Muskel (22) unterstützbaren elastischen Pumpkammer (20), wobei die Vorrichtung mit der Kammer verbundene rohrförmige Shunts, an die Shunts angekoppelte Ventile sowie mit einer elektrischen Stromquelle und mit dem Muskel verbundene Elektroden aufweist,

gekennzeichnet durch

eine Anordnung zum Pumpen von Blut von dem linken Ventrikel zu der Aorta des Herzens eines Patienten in Synchronismus mit den Ventrikeldepolarisationen, wobei die Pumpanordnung aufweist

einen mit einem proximalen und einem distalen Ende versehenen ersten rohrförmigen Shunt zur Verbindung mit dem linken Ventrikel des Herzens über das proximale Ende;

einen mit einem proximalen und einem distalen Ende versehenen zweiten rohrförmigen Shunt zur Verbindung mit der Aorta des Herzens;

wobei die elastische Pumpkammer (20) eine von einer großen Achse (26) und einer kleinen Achse (24) bestimmte abgeflachte Ellipsoidgestalt hat, von einer Hülle aus inerviertem autogenem Muskelgewebe (22) im wesentlichen vollständig umgeben werden kann sowie eine erste und eine zweite Öffnung aufweist, die am Umfang konzentrisch zu der großen Achse angeordnet sind;

ein mit dem distalen Ende des ersten rohrförmigen Shunts und mit der ersten Öffnung gekoppeltes Einrichtungs-Aorten-Ventil (28);

ein mit dem distalen Ende des zweiten rohrförmigen Shunts und mit der zweiten Öffnung gekoppeltes Einrichtungs-Mitralventil (30);

einen Impulsgenerator (36), der an eine erste (32) und eine zweite (34) Elektrode ankoppelbar ist,

- um Muskelgewebe mit chronischen Stimulationsimpulsen mit einer als Ultraniederfrequenz bezeichneten Frequenz zu versorgen, die niedrig genug ist, um eine hohe Population an langsam zuckenden Muskelfasern aufrecht zu erhalten; und

- um ferner Stimulationsimpulse in Synchronismus mit erfaßten Depolarisationen des Ventrikels mit solcher Amplitude anzuliefern, daß das Muskelgewebe (22) stimuliert wird, um die Pumpkammer (20) zusammenzupressen; wobei die erste Elektrode (32) an das autogene Muskelgewebe zwecks Stimulation ankoppelbar ist und mit dem Impulsgenerator (36) gekoppelt ist; und wobei die zweite Elektrode (34) an den Ventrikel ankoppelbar ist, um Depolarisationen von ventrikulären Geweben zu ermitteln.

2. Vorrichtung nach Anspruch 1,wobei die Kammer (20) ein Volumen hat, das größer als 100 ml aber kleiner als 200ml ist.

3. Vorrichtung nach Anspruch 1, wobei der erste rohrförmige Shunt einen Innendurchmesser zwischen 1,5 cm und 2,5 cm hat.

4. Vorrichtung nach Anspruch 1, wobei der zweite rohrförmige Shunt einen Innendurchmesser zwischen 1,1 cm und 2,1 cm hat.

5. Vorrichtung nach Anspruch 1, wobei die Längen der lotrechten kleinen Achse (b) und der waagrechten Achse (a) die Beziehung: $b = 5{,}78/a^{1/2}$ erfüllen.

6. Vorrichtung nach Anspruch 1, wobei die zum Umhüllen der Kammer erforderliche Muskelmasse, m, großer als 350g und kleiner als 600 g ist.

7. Vorrichtung nach Anspruch 1, wobei die Ultraniederfrequenz-Stimulationsimpulse Rechteckwellen sind und mit einer Frequenz erzeugt werden, die größer als 20 Hz und Kleiner als 50 Hz ist und vorzugsweise 35 Hz beträgt.

8. Vorrichtung zur Herzunterstützung mit einer von einem Muskel (22) unterstützbaren elastischen Pumpkammer (20), wobei die Vorrichtung Elektroden aufweist, die mit einer elektrischen Stromquelle und mit dem Muskel verbunden sind,

gekennzeichnet durch

eine Anordnung zum Vermehren des Blutstroms von dem linken Ventrikel zu der Aorta in Synchronismus mit der Ventrikel Repolarisation des Herzens des Patien ten,wobei die Anordnung versehen ist mit:

einer elastischen kugeligen Pumpkammer (12, 14, 16), die von einer Hülle aus innerviertem autogenem Muskelgewebe (22) umgeben ist;

wobei diese Kammer in der durchtrennten Aorta angeordnet werden kann;

einem Impulsgenerator (36), der an eine erste (32) und eine zweite (34) Elektrode ankoppelbar ist;

wobei die erste Elektrode an das autogene Muskelgewebe zwecks Stimulation sowie an den Impulsgenerator ankoppelbar ist, wodurch der Impulsgenerator für eine chronische Stimulation des Muskelgewebes mit einer als Ultraniederfrequenz bezeichneten Frequenz sorgt, die niedrig genug ist, um eine hohe Population an langsam zuckenden Muskelfasern aufrecht zu erhalten; und

wobei die zweite Elektrode an den Ventrikel ankoppelbar ist, um Endrepolarisationen von ventrikulären Geweben zu ermitteln.

9. Vorrichtung nach Anspruch 8, wobei die Kammer mit der durchtrennten Aorta über ein T-Verbindungsstück verbunden wird.

10. Vorrichtung nach Anspruch 8, wobei die Kammer mit der durchtrennten Aorta über ein U-Verbindungsstück verbunden wird.

11. Vorrichtung nach Anspruch 8,9 oder 10, wobei die Kammer ein Volumen zwischen 30 und 70 ml, und vorzugsweise 50ml, hat.

12. Vorrichtung nach Ansprüchen 8, 9, 10 oder 11, wobei Gegenpulsationen benutzt werden, um primärer oder essentieller Hypertonie zu begegnen.